# EUROPEAN PATENT SPECIFICATION

(11) **EP 3 034 172 B1**
(45) Date of publication and mention of the grant of the patent: **31.01.2018**
(21) Application number: 14844599.2
(22) Date of filing: 01.09.2014
(51) Int. Cl.: B02C 18/18, A61F 13/15, A61F 13/472, A61F 13/49, B02C 18/14

(54) **PULVERIZER, ABSORBER MANUFACTURING DEVICE PROVIDED WITH SAME, AND PULP SHEET PULVERIZING METHOD**
ZERSTÄUBER, ABSORBERHERSTELLUNGSVORRICHTUNG DAMIT UND VERFAHREN ZUR ZERSTÄUBUNG EINER ZELLSTOFFSCHICHT
PULVÉRISATEUR, DISPOSITIF DE FABRICATION D'ABSORBEUR LE COMPORTANT, ET PROCÉDÉ DE PULVÉRISATION DE FEUILLE DE PÂTE

(30) Priority: 12.09.2013 JP 2013189612
(43) Date of publication of application: 22.06.2016
(73) Proprietor: Zuiko Corporation, Settsu-shi Osaka 566-0045 (JP)
(72) Inventor: WADA, Takao, Settsu-shi Osaka 566-0045 (JP)
(74) Representative: Müller-Boré & Partner Patentanwälte PartG mbB
(86) International application number: PCT/JP2014/072932
(87) International publication number: WO 2015/037466

(56) References cited:
- JP-A- S6 434 452
- JP-A- 2005 161 289
- JP-A- 2005 161 289
- JP-A- 2007 167 822
- JP-A- 2011 152 351
- JP-B2- H0 143 338
- US-A1- 2013 014 899

## Description

### TECHNICAL FIELD

The present invention relates to a pulverizer for pulverizing a pulp sheet, and an absorber manufacturing apparatus provided with the same.

### BACKGROUND ART

An absorber for use in disposable diapers or the like is manufactured by forming pulp fibers obtained by pulverizing a pulp sheet into a predetermined shape.

A pulverizer described in Patent Literature 1 is known as an example of an apparatus for pulverizing a pulp sheet.

The pulverizer described in Patent Literature 1 is provided with a casing, and a rotary blade accommodated in the casing and including pulverizing blades rotatable around a rotary shaft.

The rotary blade pulverizes a pulp sheet introduced into the casing by rotating the pulverizing blades around the rotary shaft.

Patent Literature 1 discloses the shape of the rotary blade as viewed along the rotary shaft, but fails to disclose the configuration of the rotary blade as viewed in the direction orthogonal to the rotary shaft.

In other words, regarding the rotary blade of Patent Literature 1, there is no measure for uniformly pulverizing a pulp sheet in the axial direction of the rotary shaft.
Patent Literature 2 discloses an apparatus for manufacturing an absorbent body including: a sheet supplying mechanism for supplying a sheet from a sheet roll; a crusher for crushing the sheet into a fibrous matter by scraping a reeling-out tip end of the reeled out sheet; and a fiber stacking device for forming the absorbent body in a predetermined shape by laminating the fibrous matter to be sent out from the crusher.
Patent Literature 3 discloses an apparatus for crushing expanded polystyrene, in which spent expanded polystyrene is crushed so rationally that it is easily reused. A crusher having a plurality of disk-shaped toothed cutters fixed aslant to a columnar rotating body having a shaft in the center parallel at predetermined intervals placed in a crushing chamber arranged separately in the main body of this apparatus via a bearing so that one end of the shaft is projected to the outside of the crushing chamber.

### CITATION LIST

### PATENT LITERATURE

Patent Literature 1: Japanese Unexamined Patent Publication No. 2011-152351
Patent Literature 2: US 2013/014899 A1
Patent Literature 3: JP 2005 161289 A

### SUMMARY OF INVENTION

An object of the invention is to provide a pulverizer that enables to uniformly pulverize a pulp sheet in the axial direction of a rotary shaft, an absorber manufacturing apparatus provided with the same, and a pulp sheet pulverizing method.

In view of the above, the invention provides a pulverizer for pulverizing a pulp sheet. The pulverizer is provided with a casing into which the pulp sheet is introduced; and a rotary blade including a plurality of cutter plates accommodated in the casing, and a rotary shaft fixed to the cutter plates in a state that the rotary shaft passes through the cutter plates in a thickness direction of the cutter plates. The cutter plates are disposed away from each other at an interval in an axial direction of the rotary shaft. Each of the cutter plates includes a plurality of pulverizing blades aligned circumferentially along an outer periphery edge of the cutter plate. At least one of the cutter plates is fixed to the rotary shaft in a state that the at least one of the cutter plate is inclined with respect to a direction orthogonal to the rotary shaft.

Further, the invention provides an absorber manufacturing apparatus for manufacturing an absorber. The absorber manufacturing apparatus is provided with the pulverizer; a fiber stacking device which forms pulp fibers obtained by pulverizing by the pulverizer into a predetermined shape of the absorber; and a duct which guides the pulp fibers from the pulverizer to the fiber stacking device.

Further, the invention provides a method for pulverizing a pulp sheet with use of a pulverizer provided with a casing; and a rotary blade including a plurality of cutter plates accommodated in the casing, and a rotary shaft fixed to the cutter plates in a state that the rotary shaft passes through the cutter plates in a thickness direction of the cutter plates. Each of the cutter plates includes a plurality of pulverizing blades aligned circumferentially along an outer periphery edge of the cutter plate. The method includes a rotating step of rotating the rotary shaft in a state that the cutter plates are disposed away from each other at an interval in an axial direction of the rotary shaft, and that at least one of the cutter plates is inclined with respect to a direction orthogonal to the rotary shaft; and an introducing step of introducing the pulp sheet into the casing in a state that the rotating step is executed.

According to the invention, it is possible to uniformly pulverize a pulp sheet in the width direction of the pulp sheet.

### BRIEF DESCRIPTION OF DRAWINGS

FIG. 1 is a sectional side view schematically illustrating an overall configuration of an absorber manufacturing apparatus embodying the invention;
FIG. 2 is a sectional side view enlargedly illustrating a pulverizer illustrated in FIG. 1;
FIG. 3 is a front view partly illustrating a rotary blade illustrated in FIG. 2; and
FIG. 4 is a schematic diagram for describing tilt angles of pulverizing blades illustrated in FIG. 3.

### DESCRIPTION OF EMBODIMENTS

In the following, an embodiment of the invention is described referring to the accompanying drawings. The following embodiment is an example embodying the invention, and does not limit the technical scope of the invention.

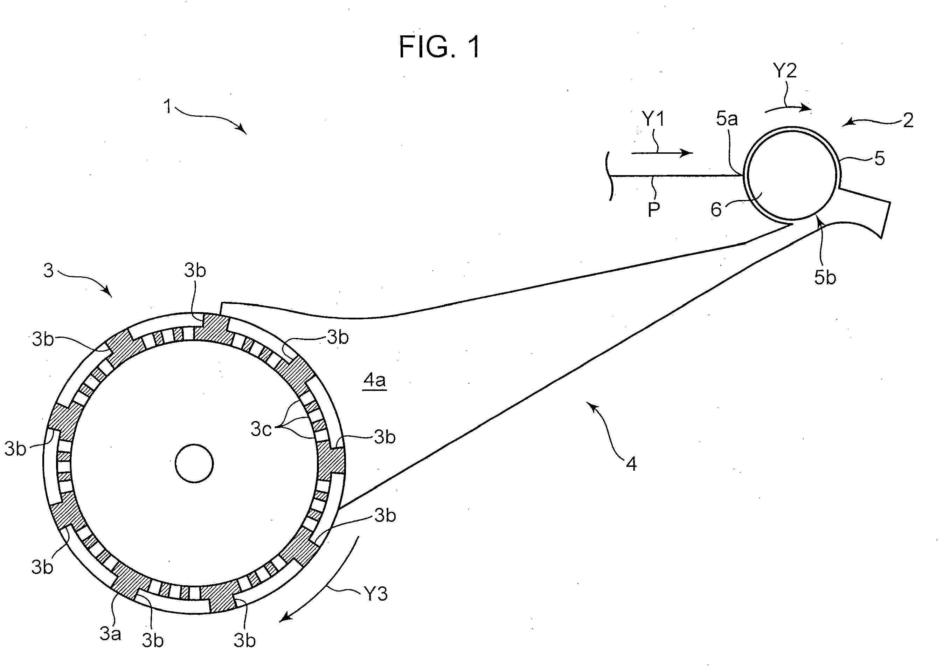

Referring to FIG. 1, an absorber manufacturing apparatus 1 is provided with a pulverizer 2 which pulverizes a pulp sheet P and generates pulp fibers, a fiber stacking device 3 which forms the pulp fibers obtained by pulverizing by the pulverizer 2 into a predetermined shape of an absorber, and a duct 4 which guides the pulp fibers from the pulverizer 2 to the fiber stacking device 3.

The duct 4 is connected to a pulp fiber outlet port 5b of the pulverizer 2.

The fiber stacking device 3 is provided with a rotary drum 3a including recess portions 3b of a shape corresponding to the shape of the absorber on the outer surface of the rotary drum 3a.

The rotary drum 3a is disposed at such a position that a part of the outer surface thereof is located in an opening 4a formed on the downstream side of the duct 4. The rotary drum 3a is rotatable in the direction of the arrow Y3 so as to allow the recess portions 3b to face the opening 4a one after another.

Further, the rotary drum 3a includes air suction ports 3c communicating with the bottom portions of the recess portions 3b. Allowing the air in the recess portions 3b to be sucked through the air suction ports 3c makes it possible to suck the pulp fibers in the duct 4 into the recess portions 3b, and to form the pulp fibers into the predetermined shape of the absorber.

In the following, an exemplified configuration of the pulverizer 2 is described referring to FIG. 1 to FIG. 3.

The pulverizer 2 is provided with a casing 5 into which the pulp sheet P is introduced in the direction indicated by the arrow Y1, a rotary blade 6 which is rotated in the direction indicated by the arrow Y2 for pulverizing the pulp sheet P in the casing 5, and a motor 7 which is driven to rotate the rotary blade 6.

The casing 5 includes an inlet port 5a which introduces the pulp sheet P, and the outlet port 5b which guides the pulp fibers obtained by pulverizing by the rotary blade 6. The inlet port 5a and the outlet port 5b are formed at positions away from each other circumferentially of a rotary shaft 8 of the rotary blade 6 to be described later.

The rotary blade 6 is provided with a plurality of cutter plates 9 accommodated in the casing 5, the rotary shaft 8 fixed to the cutter plates 9 in a state that the rotary shaft 8 passes through the cutter plates 9 in the thickness direction of the cutter plates 9, a plurality of spacers 10 disposed between the adjacent cutter plates 9, and a pair of holding members 11 which hold the cutter plates 9 from both sides in the axial direction of the rotary shaft 8.

Each of the cutter plates 9 is provided with a disc-shaped body portion 9a fixed to the rotary shaft 8, and a plurality of pulverizing blades 9b, which project radially outward of the rotary shaft 8 from the body portion 9a and which align circumferentially of the rotary shaft 8.

Further, the cutter plates 9 are disposed away from each other at an interval in the axial direction of the rotary shaft 8. The interval between the adjacent cutter plates 9 is defined by the thickness of the spacer 10. The spacer 10 is a disc-shaped member through which the rotary shaft 8 passes.

Further, all the cutter plates 9 are disposed in parallel to each other in a state that each of the cutter plates 9 is inclined by the angle θ with respect to a direction orthogonal to the rotary shaft 8. Specifically, each of the the cutter plates 9 is inclined by the angle θ while being held by the paired holding members 11 in a state that each of the spacers 10 is interposed between the adjacent cutter plates 9.

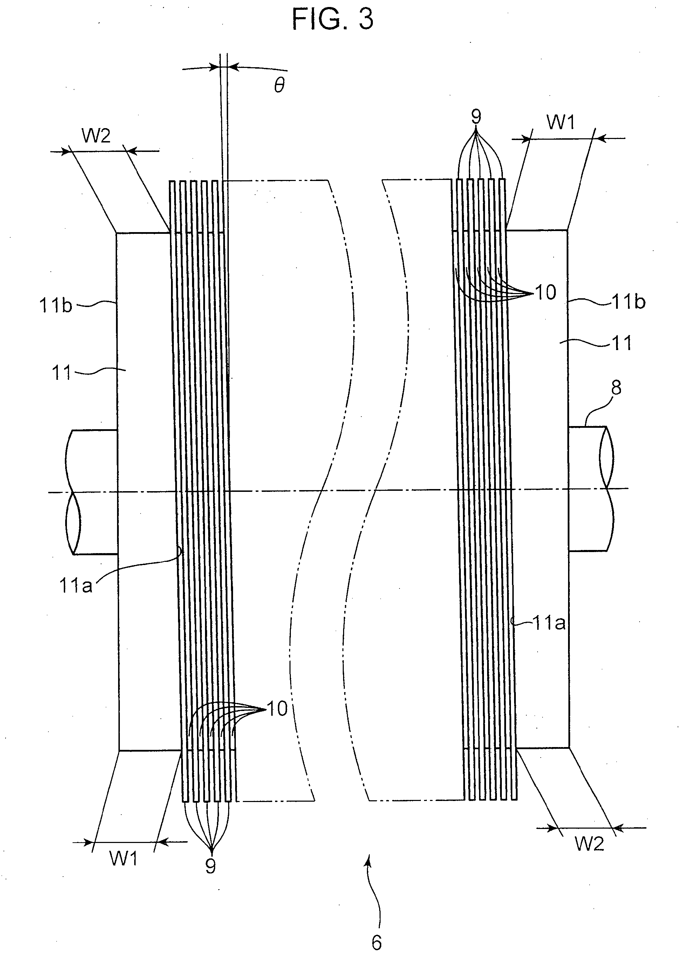

As illustrated in FIG. 3, each of the holding members 11 includes an inner surface 11a inclined by the angle θ with respect to the direction orthogonal to the rotary shaft 8 while facing the cutter plates 9, and an outer surface 11b extending in a direction orthogonal to the rotary shaft 8 while facing the side opposite to the inner surface 11a side.

In other words, each of the holding members 11 includes a thick portion having a largest thickness W1, and a thin portion formed at a position displaced from the thick portion by 180° around the rotary shaft 8 and having a smallest thickness W2.

Holding the cutter plates 9 and the spacers 10 between the paired holding members 11 in a state that the thick portion of one of the holding members 11 and the thin portion of the other of the holding members 11 face each other and that the thin portion of the one of the holding members 11 and the thick portion of the other of the holding members 11 face each other makes it possible to incline the cutter plates 9 by the angle θ with respect to the direction orthogonal to the rotary shaft 8.

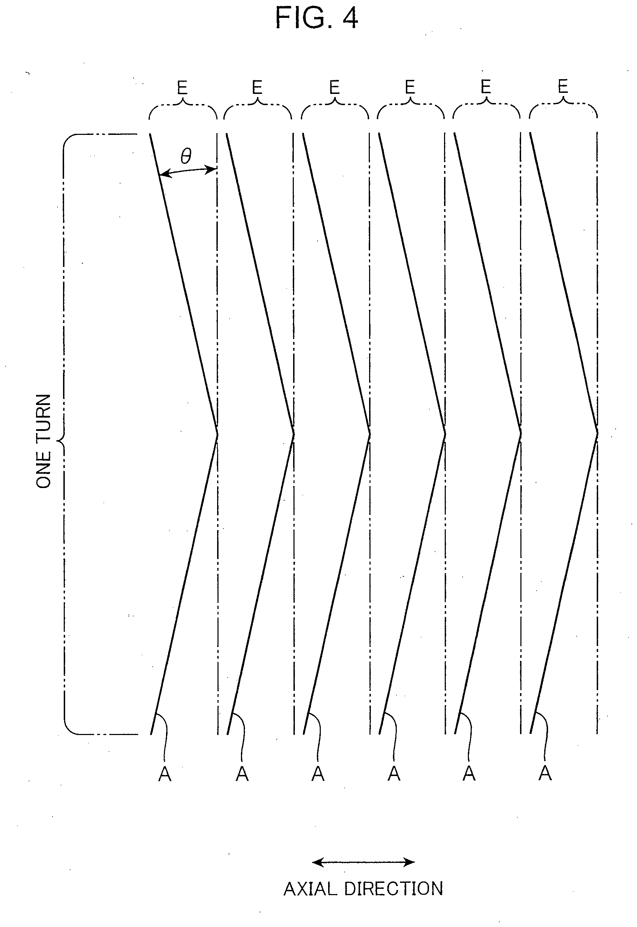

Referring to FIG. 4, the tilt angle θ of the cutter plates 9 is described. In FIG. 4, the reference sign A indicates a trajectory of the pulverizing blades 9b of the cutter plates 9 when the rotary shaft 8 makes one turn.

The tilt angle θ of a specific one of the cutter plates 9 (e.g. see the leftmost trajectory A in FIG. 4) with respect to the rotary shaft 8 is set in such a manner that the pulverizing range E by which the trajectory A is axially displaced when the rotary shaft 8 makes one turn is deviated from the pulverizing range E of the cutter plate 9 adjacent to the specific cutter plate 9 (e.g. see the second leftmost trajectory A in FIG. 4).

Each of the cutter plates 9 has an unillustrated key groove communicating with a through-hole through which the rotary shaft 8 passes. Engaging a key formed on the rotary shaft 8 in the key groove restricts relative rotation between the cutter plate 9 and the rotary shaft 8.

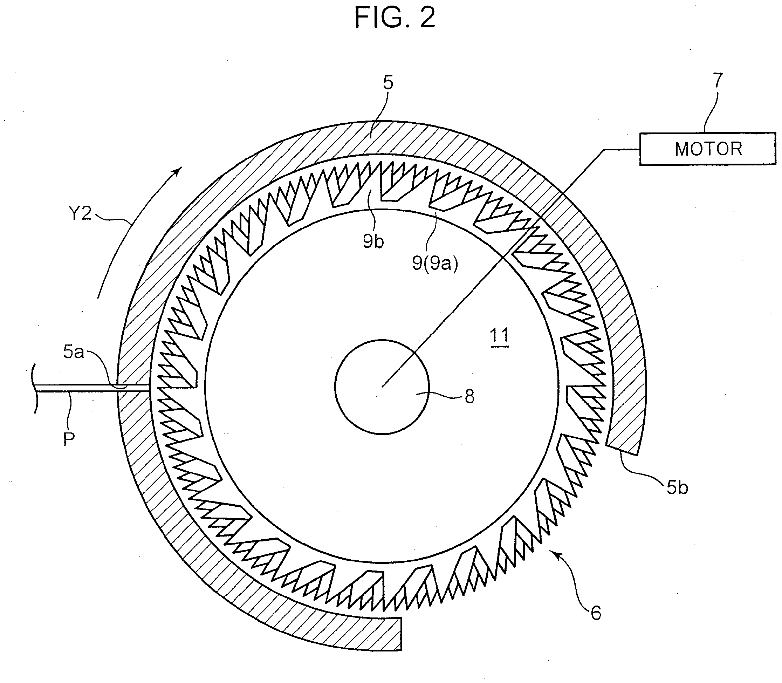

Respective two adjacent cutter plates 9 of the cutter plates 9 are disposed such that the circumferential positional relationship between the key groove and the pulverizing blade 9b is different from each other. Therefore, as illustrated in FIG. 2, the pulverizing blades 9b of the respective two adjacent cutter plates 9 are disposed away from each other circumferentially of the rotary shaft 8.

Further, the paired holding members 11 are axially fixed to the rotary shaft 8 by unillustrated bolts. Therefore, the cutter plates 9 held between the paired holding members 11 are also axially fixed to the rotary shaft 8.

When the pulp sheet P is pulverized with use of the pulverizer 2, first of all, the rotary shaft 8 is rotated in the direction indicated by the arrow Y2 in FIG. 1 (a rotating step).

Specifically, in the rotating step, as illustrated in FIG. 3, the rotary shaft 8 is rotated in a state that the plurality of cutter plates 9 are disposed away from each other in the axial direction of the rotary shaft 8, and that all the cutter plates 9 are inclined with respect to the direction orthogonal to the rotary shaft 8.

In a state that the rotating step is executed, the pulp sheet P is introduced into the casing 5 radially from the outside of the rotary shaft 8 (cutter plates 9) through the inlet port 5a along the direction indicated by the arrow Y1 in FIG. 1 (an introducing step).

Then, as illustrated in FIG. 2, the pulp sheet P comes into contact with the pulverizing blades 9b of the cutter plates 9 and is pulverized.

As described above, the cutter plates 9 are fixed to the rotary shaft 8 in a state that the cutter plates 9 are spaced away from each other in the axial direction of the rotary shaft 8. Therefore, it is possible to uniformly abut the pulverizing blades of the cutter plates 9 against the pulp sheet P in the axial direction of the rotary shaft 8 by rotating the rotary shaft 8.

Further, at least one of the cutter plates 9 is inclined with respect to the direction orthogonal to the rotary shaft 8. Therefore, the abutting positions of the pulverizing blades 9b of the inclined cutter plate 9 against the pulp sheet P are axially displaced, as the rotary shaft 8 is rotated.

Thus, according to the invention, it is possible to uniformly pulverize the pulp sheet P in the axial direction of the rotary shaft 8.

Further, the embodiment provides the following advantageous effects.

The abutting positions of the pulverizing blades 9b of all the cutter plates 9 against the pulp sheet P are axially displaced, as the rotary shaft 8 is rotated. This is advantageous in uniformly pulverizing the pulp sheet P in the axial direction of the rotary shaft 8.

Unlike a configuration, in which the tilt angles of the cutter plates 9 with respect to the rotary shaft 8 are different from each other, disposing the cutter plates 9 in parallel to each other makes it possible to eliminate a variation in the distance by which the pulverizing blades 9b of the respective two adjacent cutter plates 9 are axially away from each other, and makes it possible to keep the largest axial distance between the pulverizing blades 9b of the respective two adjacent cutter plates 9 small.

Thus, the aforementioned configuration is advantageous in uniformly pulverizing the pulp sheet P in the axial direction of the rotary shaft 8.

The tilt angle θ of the cutter plates 9 is set in such a manner that the pulverizing ranges E of the adjacent cutter plates 9 do not overlap each other. According to this configuration, the pulverizing blades 9b of the adjacent cutter plates 9 pass the ranges different from each other in the axial direction of the rotary shaft 8. This is advantageous in efficiently pulverizing the pulp sheet P with use of all the pulverizing blades 9b.

The pulverizing blades 9b of the respective two adjacent cutter plates 9 are disposed at positions away from each other circumferentially of the rotary shaft 8. Therefore, unlike a configuration, in which the pulverizing blades 9b of the adjacent cutter plates 9 are simultaneously abutted against the pulp sheet P, it is possible to exert a local force on the pulp sheet P by abutting the pulverizing blades 9b against the pulp sheet P one after another.

Thus, the aforementioned configuration is advantageous in finely pulverizing the pulp sheet P in the axial direction of the rotary shaft 8.

It should be noted that the following modifications are applicable.

It is possible to incline at least one of the cutter plates 9 with respect to the direction orthogonal to the rotary shaft 8, in place of inclining all the cutter plates 9.

It is possible to incline the cutter plates 9 with respect to the rotary shaft 8 at different angles from each other, in place of aligning the cutter plates 9 in parallel to each other.

It is possible to set the tilt angles of the cutter plates 9 in such a manner that the pulverizing ranges E of the respective two adjacent cutter plates 9 overlap each other.

It is possible to dispose the pulverizing blades 9b of the respective two adjacent cutter plates 9 at the same position circumferentially of the rotary shaft 8.

The specific embodiments described above mainly include the invention having the following configurations.

Specifically, the invention provides a pulverizer for pulverizing a pulp sheet. The pulverizer is provided with a casing into which the pulp sheet is introduced; and a rotary blade including a plurality of cutter plates accommodated in the casing, and a rotary shaft fixed to the cutter plates in a state that the rotary shaft passes through the cutter plates in a thickness direction of the cutter plates. The cutter plates are disposed away from each other at an interval in an axial direction of the rotary shaft. Each of the cutter plates includes a plurality of pulverizing blades aligned circumferentially along an outer periphery edge of the cutter plate. At least one of the cutter plates is fixed to the rotary shaft in a state that the at least one of the cutter plate is inclined with respect to a direction orthogonal to the rotary shaft.

According to the invention, the cutter plates are fixed to the rotary shaft in a state that the cutter plates are disposed away from each other at an interval in the axial direction of the rotary shaft. Therefore, it is possible to uniformly abut the pulverizing blades of the cutter plates against the pulp sheet in the axial direction of the rotary shaft by rotating the rotary shaft.

Further, at least one of the cutter plates is inclined with respect to the direction orthogonal to the rotary shaft. Therefore, the abutting positions of the pulverizing blades of the cutter plate against the pulp sheet are axially displaced, as the rotary shaft is rotated.

Thus, according to the invention, it is possible to uniformly pulverize the pulp sheet in the axial direction of the rotary shaft.

In the pulverizer, preferably, all the cutter plates may be inclined with respect to the direction orthogonal to the rotary shaft.

According to the aforementioned configuration, the abutting positions of the pulverizing blades of all the cutter plates against the pulp sheet are axially displaced, as the rotary shaft is rotated. This is advantageous in uniformly pulverizing the pulp sheet in the axial direction of the rotary shaft.

In the aforementioned configuration, the tilt angles of the cutter plates with respect to the rotary shaft may be set at different angles from each other. In the pulverizer, preferably, the cutter plates may be disposed in parallel to each other.

When the tilt angles of the cutter plates with respect to the rotary shaft are set at different angles from each other, the distance by which the pulverizing blades of the respective two adjacent cutter plates are axially away from each other may vary, as the rotary shaft is rotated, and the largest axial distance between the pulverizing blades of the respective two adjacent cutter plates may be large.

Contrary to the above, according to the aforementioned configuration, the cutter plates are disposed in parallel to each other. This makes it possible to eliminate a variation in the distance by which the pulverizing blades of the respective two adjacent cutter plates are axially away from each other, and makes it possible to keep the largest axial distance between the pulverizing blades of the respective adjacent cutter plates small.

Thus, the aforementioned configuration is advantageous in uniformly pulverizing the pulp sheet in the axial direction of the rotary shaft.

In the pulverizer, preferably, a tilt angle of a specific one of the cutter plates with respect to the rotary shaft may be set in such a manner that a pulverizing range by which the pulverizing blades of the specific cutter plate are axially displaced when the rotary shaft makes one turn is deviated from a pulverizing range of the cutter plate adjacent to the specific cutter plate.

When the pulverizing ranges of the adjacent cutter plates overlap each other, the pulverizing blades of the adjacent cutter plates pass the same range in the axial direction of the rotary shaft. This may lower the pulverizing efficiency of the pulp sheet by the pulverizing blades.

Contrary to the above, according to the aforementioned configuration, the tilt angles of the cutter plates are set in such a manner that the pulverizing ranges of the adjacent cutter plates do not overlap each other. This allows for the pulverizing blades of the adjacent cutter plates to pass the ranges different from each other in the axial direction of the rotary shaft. This is advantageous in efficiently pulverizing the pulp sheet with use of all the pulverizing blades.

In the pulverizer, preferably, the pulverizing blades of the respective two adjacent cutter plates of the cutter plates may be disposed at positions away from each other circumferentially of the rotary shaft.

According to the aforementioned configuration, as compared with a configuration, in which the pulverizing blades of the adjacent cutter plates are simultaneously abutted against the pulp sheet, it is possible to exert a local force on the pulp sheet by abutting the pulverizing blades against the pulp sheet one after another.

Thus, the aforementioned configuration is advantageous in finely pulverizing the pulp sheet in the axial direction of the rotary shaft.

Further, the invention provides an absorber manufacturing apparatus for manufacturing an absorber. The absorber manufacturing apparatus is provided with the pulverizer; a fiber stacking device which forms pulp fibers obtained by pulverizing by the pulverizer into a predetermined shape of the absorber; and a duct which guides the pulp fibers from the pulverizer to the fiber stacking device.

Further, the invention provides a method for pulverizing a pulp sheet with use of a pulverizer provided with a casing; and a rotary blade including a plurality of cutter plates accommodated in the casing, and a rotary shaft fixed to the cutter plates in a state that the rotary shaft passes through the cutter plates in a thickness direction of the cutter plates. Each of the cutter plates includes a plurality of pulverizing blades aligned circumferentially along an outer periphery edge of the cutter plate. The method includes a rotating step of rotating the rotary shaft in a state that the cutter plates are disposed away from each other at an interval in an axial direction of the rotary shaft, and that at least one of the cutter plates is inclined with respect to a direction orthogonal to the rotary shaft; and an introducing step of introducing the pulp sheet into the casing in a state that the rotating step is executed.

According to the invention, the rotary shaft is fixed in a state that the cutter plates are disposed away from each other at an interval in the axial direction of the rotary shaft. Therefore, it is possible to uniformly abut the pulverizing blades of the cutter plates against the pulp sheet in the axial direction of the rotary shaft by rotating the rotary shaft.

Further, at least one of the cutter plates is inclined with respect to the direction orthogonal to the rotary shaft. Therefore, it is possible to axially displace the abutting positions of the pulverizing blades of the cutter plate, as the rotary shaft is rotated.

Thus, according to the invention, it is possible to uniformly pulverize the pulp sheet in the axial direction of the rotary shaft.

## Claims

1. A pulverizer for pulverizing a pulp sheet, comprising:
a casing into which the pulp sheet is introduced; and
a rotary blade including a plurality of cutter plates accommodated in the casing, and a rotary shaft fixed to the cutter plates in a state that the rotary shaft passes through the cutter plates in a thickness direction of the cutter plates, wherein
the cutter plates are disposed away from each other at an interval in an axial direction of the rotary shaft,
each of the cutter plates includes a plurality of pulverizing blades aligned circumferentially along an outer periphery edge of the cutter plate,
all the cutter plates are fixed to the rotary shaft in a state that all the cutter plates are inclined with respect to a direction orthogonal to the rotary shaft and disposed in parallel to each other,
a tilt angle of a specific one of the cutter plates with respect to the rotary shaft is set in such a manner that a pulverizing range by which the pulverizing blades of the specific cutter plate are axially displaced when the rotary shaft makes one turn and a pulverizing range of the cutter plate adjacent to the specific cutter plate overlap each other, and
the pulverizing blades of the respective two adjacent cutter plates of the cutter plates are disposed at positions away from each other circumferentially of the rotary shaft.

2. An absorber manufacturing apparatus for manufacturing an absorber, comprising:
the pulverizer of claim 1;
a fiber stacking device which forms pulp fibers obtained by pulverizing by the pulverizer into a predetermined shape of the absorber; and
a duct which guides the pulp fibers from the pulverizer to the fiber stacking device.

3. A method for pulverizing a pulp sheet with use of a pulverizer provided with a casing; and a rotary blade including a plurality of cutter plates accommodated in the casing, and a rotary shaft fixed to the cutter plates in a state that the rotary shaft passes through the cutter plates in a thickness direction of the cutter plates, each of the cutter plates including a plurality of pulverizing blades aligned circumferentially along an outer periphery edge of the cutter plate, the method comprising:
a rotating step of rotating the rotary shaft in a state that the cutter plates are disposed away from each other at an interval in an axial direction of the rotary shaft, that all the cutter plates are inclined with respect to a direction orthogonal to the rotary shaft and disposed in parallel to each other, that a tilt angle of a specific one of the cutter plates with respect to the rotary shaft is set in such a manner that a pulverizing range by which the pulverizing blades of the specific cutter plate are axially displaced when the rotary shaft makes one turn and a pulverizing range of the cutter plate adjacent to the specific cutter plate overlap each other, and the pulverizing blades of the respective two adjacent cutter plates of the cutter plates are disposed at positions away from each other circumferentially of the rotary shaft; and
an introducing step of introducing the pulp sheet into the casing in a state that the rotating step is executed.

## Patentansprüche

1. Zerkleinerer zum Zerkleinern eines Zellstoffbogens, umfassend:
ein Gehäuse, in das der Zellstoffbogen eingeführt wird; und
eine Drehklinge, die eine Vielzahl von Schneidplatten umfasst, die in dem Gehäuse aufgenommen sind, und eine Drehwelle, die an den Schneidplatten in einem Zustand befestigt ist, in dem die Drehwelle durch die Schneidplatten in einer Dickenrichtung der Schneidplatten hindurchgeht, wobei
die Schneidplatten entfernt voneinander in einem Intervall in einer axialen Richtung der Drehwelle angeordnet sind,
jede der Schneidplatten eine Vielzahl von Zerkleinerungsklingen umfasst, die um den Umfang entlang einer äußeren Randkante der Schneidplatte ausgerichtet sind,
alle Schneidplatten an der Drehwelle befestigt sind, in einem Zustand, in dem alle Schneidplatten in Bezug auf eine Richtung, die rechtwinklig zur Drehwelle ist, geneigt und parallel zueinander angeordnet sind,
ein Kippwinkel einer bestimmten der Schneidplatten in Bezug auf die Drehwelle in einer solchen Weise eingestellt ist, dass ein Zerkleinerungsbereich, um den die Zerkleinerungsklingen der bestimmten Schneidplatte axial versetzt sind, wenn die Drehwelle eine Umdrehung ausführt, und ein Zerkleinerungsbereich der Schneidplatte, die benachbart zu der bestimmten Schneidplatte ist, sich gegenseitig überlappen, und
die Zerkleinerungsklingen der jeweils zwei benachbarten Schneidplatten der Schneidplatten in Positionen angeordnet sind, die entlang dem Umfang der Drehwelle entfernt voneinander sind.

2. Absorberherstellungseinrichtung zum Herstellen eines Absorbers, umfassend:
einen Zerkleinerer nach Anspruch 1;
eine Faserstapelvorrichtung, die Zellstofffasern bildet, die durch das Zerkleinern durch den Zerkleinerer erhalten werden, in eine vorbestimmte Form des Absorbers; und
eine Leitung, die die Zellstofffasern vom Zerkleinerer zur Faserstapelvorrichtung führt.

3. Verfahren zum Zerkleinern eines Zellstoffbogens unter Verwendung eines Zerkleinerers, der mit einem Gehäuse versehen ist; und eine Drehklinge, die eine Vielzahl von Schneidplatten umfasst, die in dem Gehäuse aufgenommen sind, und eine Drehwelle, die an den Schneidplatten befestigt ist, in einem Zustand, in dem die Drehwelle durch die Schneidplatten in einer Dickenrichtung der Schneidplatten hindurchgeht, wobei jede der Schneidplatten eine Vielzahl von Zerkleinerungsklingen umfasst, die um den Umfang entlang einer äußeren Randkante der Schneidplatte ausgerichtet sind, wobei das Verfahren Folgendes umfasst:
einen Drehschritt des Drehens der Drehwelle in einem Zustand, in dem die Schneidplatten entfernt voneinander in einem Intervall in einer axialen Richtung der Drehwelle angeordnet sind, in dem alle Schneidplatten in Bezug auf eine Richtung, die rechtwinklig zur Drehwelle ist, geneigt und parallel zueinander angeordnet sind, in dem ein Kippwinkel einer bestimmten der Schneidplatten in Bezug auf die Drehwelle in einer solchen Weise eingestellt ist, dass ein Zerkleinerungsbereich, um den die Zerkleinerungsklingen der bestimmten Schneidplatte axial versetzt sind, wenn die Drehwelle eine Umdrehung ausführt, und ein Zerkleinerungsbereich der Schneidplatte, die benachbart zu der bestimmten Schneidplatte ist, sich gegenseitig überlappen, und die Zerkleinerungsklingen der jeweils zwei benachbarten Schneidplatten der Schneidplatten in Positionen angeordnet sind, die entlang dem Umfang der Drehwelle entfernt voneinander sind; und
einen Einführschritt des Einführens des Zellstoffbogens in das Gehäuse in einem Zustand, in dem der Drehschritt ausgeführt wird.

## Revendications

1. Pulvérisateur destiné à pulvériser une feuille de pâte comportant :
un boîtier dans lequel la feuille de pâte est introduite ; et
une lame rotative comprenant une pluralité de plaques de couteaux logées dans le boîtier et un arbre rotatif fixé sur les plaques de couteaux dans un état dans lequel l'arbre rotatif passe à travers les plaques de couteaux dans une direction d'épaisseur des plaques de couteaux, dans lequel
les plaques de couteaux sont disposées éloignées l'une de l'autre selon un intervalle dans une direction axiale de l'arbre rotatif,
chacune des plaques de couteaux comprend une pluralité de lames de pulvérisation alignées de façon circonférentielle le long d'un bord périphérique extérieur de la plaque de couteaux,
toutes les plaques de couteaux sont fixées sur l'arbre rotatif dans un état dans lequel toutes les plaques de couteaux sont inclinées par rapport à une direction orthogonale à l'arbre rotatif et disposées parallèlement les unes aux autres,
un angle d'inclinaison d'une plaque de couteaux spécifique, parmi les plaques de couteaux, par rapport à l'arbre rotatif est défini de manière à ce qu'une plage de pulvérisation, selon laquelle les lames de pulvérisation de la plaque de couteaux spécifique sont déplacées axialement lorsque l'arbre rotatif effectue un tour, et une plage de pulvérisation de la plaque de couteaux adjacente à la plaque de couteaux spécifique se chevauchent l'une l'autre et
les lames de pulvérisation des deux plaques de couteaux adjacentes respectives, parmi les plaques de couteaux,
sont disposées selon des positions opposées l'une à l'autre au plan circonférentiel par rapport à l'arbre rotatif.

2. Appareil de fabrication d'absorbeur destiné à fabriquer un absorbeur, comportant :
le pulvérisateur selon la revendication 1 ;
un dispositif d'empilement de fibres qui forme des fibres de pâte obtenues par pulvérisation à l'aide du pulvérisateur selon une forme prédéfinie de l'absorbeur ; et
une conduite qui guide les fibres de pâte, du pulvérisateur vers le dispositif d'empilement de fibres.

3. Procédé de pulvérisation d'une feuille de pâte à l'aide d'un pulvérisateur doté d'un boîtier ; et d'une lame rotative comprenant une pluralité de plaques de couteaux logées dans le boîtier, ainsi qu'un arbre rotatif fixé aux plaques de couteaux dans un état dans lequel l'arbre rotatif passe à travers les plaques de couteaux dans une direction d'épaisseur des plaques de couteaux, chacune des plaques de couteaux comprenant une pluralité de lames de pulvérisation alignées au plan circonférentiel le long d'un bord périphérique extérieur de la plaque de couteaux, le procédé comportant :
une étape de rotation consistant à faire tourner l'arbre rotatif dans un état dans lequel les plaques de couteaux sont disposées à l'opposé l'une de l'autre selon un intervalle dans une direction axiale de l'arbre rotatif, dans lequel toutes les plaques de couteaux sont inclinées par rapport à une direction orthogonale à l'arbre rotatif et disposées parallèlement l'une à l'autre, selon lequel un angle d'inclinaison d'une plaque de couteaux spécifique, parmi les plaques de couteaux, par rapport à l'arbre rotatif est défini de manière à ce qu'une plage de pulvérisation, selon laquelle les lames de pulvérisation de la plaque de couteaux spécifique sont déplacées axialement lorsque l'arbre rotatif effectue un tour, et une plage de pulvérisation de la plaque de couteaux adjacente à la plaque de couteaux spécifique se chevauchent l'une l'autre et les lames de pulvérisation des deux plaques de couteaux adjacentes respectives, parmi les plaques de couteaux, sont disposées selon des positions éloignées l'une de l'autre au plan circonférentiel par rapport à l'arbre rotatif ; et
une étape d'introduction consistant à introduire la feuille de pâte dans le boîtier, dans un état dans lequel l'étape de rotation est exécutée.
